# EUROPEAN PATENT SPECIFICATION

(11) **EP 0 422 302 B1**
(45) Date of publication and mention of the grant of the patent: **15.09.1993**
(21) Application number: 89310505.6
(22) Date of filing: 13.10.1989
(51) Int. Cl.: A61F 2/12

(54) **Implantable prosthesis**
Implantierbare Prothese
Prothèse implantable

(43) Date of publication of application: 17.04.1991
(73) Proprietor: Muller, Guy-Henri, F-67000 Strasbourg (FR)
(72) Inventor: Muller, Guy-Henri, F-67000 Strasbourg (FR)
(74) Representative: Bossard, Jacques-René

(56) References cited:
- EP-A- 0 115 384
- FR-A- 2 448 892
- GB-A- 2 047 101
- US-A- 3 559 214

## Description

This invention relates to a prosthesis which is capable of being temporarily or permanently inserted or implanted in the human body in order to fill in some irregularities, to increase the volume and/or to modify the external shape thereof.

A typical application of such prosthesis is in mammoplasty where the prosthesis takes the form of a mammary implant. A prosthesis according to the features of the preamble of Claim 1 is known from EP-A-0 115 384. The following description will be directed to such implants as a non-limitative aspect of the invention.

It is well-known that the present mammary prostheses are generally made of an envelope of flexible silicone film, having a more or less flattened spherical shape, which is filled with a silicone gel, a physiological salt solution or both. A disadvantage to the use of silicone gel is the risk of leakage of the gel through the flexible silicone film by transudation, or the loss of gel caused by rupture or tearing of said film. This sometimes results in reabsorption of the gel by the body with possible local or even general complications. A disadvantage to the use of a physiological salt solution is the risk of loss of solution due to tearing or rupture of the flexible silicone film or due to leaks, e.g. at the filling valve. Although this does not have negative physiological consequences, a decrease of the volume of the prosthesis occurs resulting in an aesthetically unacceptable appearance necessitating further surgery.

In an attempt to avoid such disadvantages prostheses comprising a central portion filled with silicone gel and a peripheral portion filled with physiological solution have been suggested in order to hinder the transudation of the gel. In fact such composite prostheses suffer from all the drawbacks described above.

Prostheses have also been proposed which comprise means of progressive temporary or permanent expansion effected by injection of e.g. saline solution in situ during surgery or over a longer or shorter period in order, for example, to induce skin development (tissue expansion) in the area concerned. However, in addition to the above mentioned drawbacks the use of such prostheses involves several surgical interventions in order to obtain a permanent implant or to remove the filling valve.

It should also be remembered that the insertion of an implant into an organism results in the spontaneous creation, around said implant, of an exclusion membrane which can be retractile and which can cause the formation of a hard capsule, especially in the case of a mammary implant (capsule retraction phenomenon). Such capsule necessitates surgical intervention for its removal.

The object of the present invention is to provide a prosthesis which overcomes the above mentioned disadvanages. This is achieved by using a new structure which avoids the use of a silicone gel and which prevents the risk of loss or leakage of the physiologial solution. Furthermore the new structure allows the modification of the volume of the prosthesis as desired so as, for example, to obtain a permanent implant without the need for any further surgical intervention.

According to the invention there is provided a prosthesis comprising a peripheral silicone membrane which is at least partially filled with a physiological salt solution characterised in that the volume delimited by said membrane is filled with a number of small individual closed cells, each delimited by a relatively thin silicone membrane and filled with a bio-compatible fluid, said small cells being distributed around a central compartment delimited by a closed, relatively thick silicone membrane, said compartment being filled with an adjustable quantity of said physiological solution.

The peripheral silicone membrane is preferably comparatively thin, for example 1 to 5mm, in order to be flexible, deformable and adaptable to varying shapes. Silicone compositions which are useful for the preparation of such membranes are well-known in the art and have been described in the literature. The membrane may comprise a thicker portion which can be punched by a needle, e.g. to inject physiological solution into the prosthesis, said thicker portion being a self-sealing silicone film. According to a modification of the invention the peripheral silicone membrane or film can be made of a self-sealing flexible silicone film in order to allow expansion of the overall volume of the prosthesis. In addition said peripheral membrane may comprise one or more rigidified portions, e.g. portions made of thick strips of silicone, with a view to give the filled prosthesis a different shape e.g. a more flattened spherical shape due to the relative resistance by these strips to lateral pressure.

The small individual closed cells which fill the peripheral silicone membrane are of a hermetic nature, are delimited by a thin membrane, e.g. having a thickness of 0.1 to 1mm, and are filled with a fluid such as air, another bio-compatible gas, a physiological salt solution or another bio-compatible liquid. When the content of the small cells is for example a physiological solution the cells will be incompressible. On the other hand, if the small cells are filled with air they will be somewhat 'elastic' in nature. Due to the hermetic nature of each small cell the assembly of these cells cannot be simply drained of its content. A substantial loss of fluid can therefore not happen accidentally. It is, however, true that punching a needle through the prosthesis in order to reach the central compartment will rupture some of the individual small cells but the resulting loss of fluid is small and of little significance due to the limited size of these cells. It is consequently preferred that each small individual closed cell should be as small as possible and most preferably have a volume smaller than 1cm³. The invention accordingly also covers the use as small cells of microcapsules well-known e.g., in the art of carbonless copying and in the pharmaceutical field.

According to a modification of the invention the assembly of small individual closed cells can comprise a silicone foam consisting of closed alveoli. Commercially available silicone foams tend to comprise a spongy mass of open alveoli which are intercommunicating. Such silicone foams are not acceptable as in case of leakage the entire mass would be drained of the fluid and the structure comprising such mass might collapse. In the modification of the invention using the silicone foam consisting of closed alveoli however, it might prove difficult to fill the closed alveoli with the bio-compatible fluid. This could be achieved e.g. by polymerisation of the foam in an aqueous medium or by dipping the already polymerised foam into an aqueous medium under very high pressure or by any other means which does not open the alveoli.

The central compartment has a silicone membrane which is at least in part similar in nature to the part of the peripheral membrane which is made of self-sealing flexible silicone. Preferably the silicone membrane of the central compartment is made of two hemispheres, one hemisphere being made of self-sealing needle punchable material, e.g. a thick silicone wall or any other anti-leakage material whilst the other hemisphere is made of extendible silicone film allowing variation of the volume of the central compartment and hence of the prosthesis. The central compartment is filled with a physiological solution. The self-sealing needle punchable part of the compartment allows the addition to, or extraction from, the central compartment of physiological solution, e.g. during a surgical intervention.

The advantages of the structure of the prostheses according to the invention are clear and include the fact that in case of accidental rupture or tearing of the peripheral membrane of the prosthesis, or of non-closing of said membrane after puncturing it with a needle for inflation or deflation of the prosthesis, only the ruptured small cells are emptied of their contents whilst the remaining untouched small cells help to retain the overall shape and volume of the unit. The well-known phenomenon of 'waves' displayed by the prior art mammary prosthesis is thereby prevented. Due to the presence of the central compartment it is possible to adjust the volume of the prosthesis, both during and after, a surgical intervention by simply inserting into the prosthesis a needle in order to extract or inject the requested amount of physiological solution from, or into, the central compartment. Such adjustment may be done with a single extraction or injection or with a number of consecutive extractions and/or injections. The prosthesis usually has an initial volume which can be adjusted during a surgical operation directly through the peripheral membrane into the central compartment, or after the operation through e.g. the breast into the prosthesis. The advantage lies therefore in the possibility of modifying the volume of the prosthesis without the need for extra surgical intervention.

A practical application is, for example, the combating of the capsule retraction phenomenon. This may be achieved by hyperinflation of the prosthesis at the time of the implantation, followed by a decrease of the volume after some weeks. If the capsule formation occurs at a later stage the prosthesis can be inflated at that time by transcutaneous punching, and decreasing its volume upon cessation of the capsule retraction phenomenon, without the need for further surgery. Another application lies in the use of the prosthesis as a valveless tissue-expansion system which is especially useful in the case of mammary reconstruction when it is desired by distension and growth of new thoracic skin to recreate local conditions on obtaining a new breast. In this case the prosthetic implant may be distended by transcutaneous punchings, followed by a further punching when the cutaneous state is found to be satisfactory in order to reach the desired mammary volume.

The prostheses according to the invention may alternatively, or additionally, comprise peripheral compartments such that the prosthesis consists of several portions which can be inflated or deflated independently. This modification allows the accommodation of the prosthesis to specific or complex thoracic distortions or to the distension of a given portion of the prosthesis, for example the lower portion of a mammary implant in the process of breast reconstruction.

In a further modification a peripheral compartment, which may be located around the peripheral silicone membrane, is provided with facets which swell when the compartment is pressurised by injection of physiological solution. Said facets give rise to discontinuities in the peripheral silicone membrane or film of the prosthesis, thus minimising the contractile possibilities of a periprosthetic capsule. This peripheral compartment may be inflated and deflated by transcutaneous punching.

In order to ensure coherence of the prosthesis comprising a central compartment and many small volumes it is preferred to have the different parts coated with an adhesive, e.g. a silicone adhesive, thereby joining each part to its neighbour.

Prostheses according to the invention may have different shapes according to the intended use and the region of the body in which they are to be inserted or implanted, for example breast, leg, chin and scalp. In each case the prosthesis provides the main advantage of adaptability during, and particularly after, an operation without necessitating further surgery.

Prostheses according to the invention may be provided in an aqueous medium, for example in a bag of physiological solution. This would avoid any dehydration of the prosthesis and also prevent any direct contact with the surgeon's hands when the prosthesis is inserted.

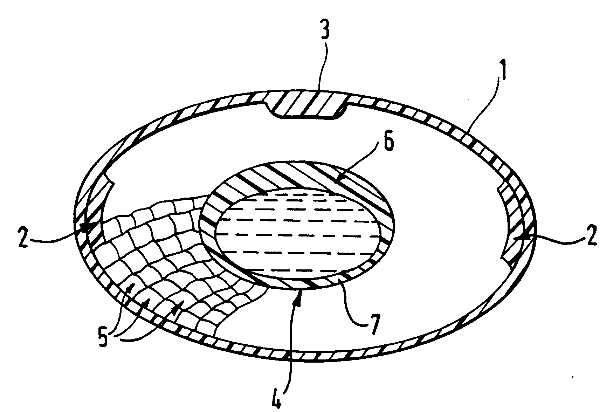

There now follows a description which is to be read in conjunction with the accompanying drawing of a prosthesis according to the invention adapted for use as a mammary implant. The drawing is a section of a mammary prosthesis.

The prosthesis is delimited by the peripheral silicone membrane (1). This membrane (1) is relatively thin, 1 to 5mm, in order to maintain its flexibility and variable shape. It comprises equatorial portions (2) which are comparatively thicker, 5 to 10mm, in order to make them more rigid, thus imparting a flattened spheroidal shape to the prosthesis. A portion (3) of the membrane (1) has a self-sealing needle punchable nature in order to allow the injection or extraction of physiological solution from the prosthesis. This portion (3) is relatively thicker than the rest of membrane (1), 2 to 5mm.

Central to the prosthesis is a central compartment (4) which comprises a membrane with an upper portion (6), the thickness of which is such that the membrane is self-sealing when punctured by an injection needle and a lower portion (7), which is thinner than (6), in order to be extensible to allow the implant to change its volume or shape.

Between the membrane (1) and the central compartment (4) are a great number of small closed volumes (5) each being hermetically delimited by a thin membrane, 0.1 to 1mm. These volumes (5) may be filled with air, a physiological solution or any other bio-compatible gas or liquid medium. Each of the volumes (5) has a volume which is preferably smaller than 1cm³ the size being compatible with the process for filling the volumes (5).

## Claims

1. A prosthesis comprising a peripheral silicone membrane (1) which is at least partially filled with a physiological salt solution characterised in that the volume delimited by said membrane (1) is filled with a number of small individual closed cells (5), each delimited by a relatively thin silicone membrane and filled with a biocompatible fluid, said small cells (5) being distributed around a central compartment (4), delimited by a closed, relatively thick silicone membrane (6,7), said compartment being filled with a constantly adjustable amount of said physiological solution.

2. A prosthesis according to Claim 1 characterised in that the said peripheral membrane (1) comprises a thicker portion (3) which is self-sealing after being punched by a needle.

3. A prosthesis according to either Claim 1 or 2 characterised in that the peripheral membrane (1) comprises one or more rigidified portions (2).

4. A prosthesis according to any one of the preceding claims characterised in that the small cells (5) have a volume of less than 1cm³.

5. A prosthesis according to any one of the preceding claims characterised in that the small cells (5) and the central compartment (4) are coated with a silicone adhesive.

6. A prosthesis according to any one of the preceding claims characterised in that the central compartment (4) comprises two hemispheres one being made of self-sealing silicone material, the other being made of extendible silicone film.

7. A prosthesis according to any one of the preceding claims characterised in that the assembly of small cells (5) comprises a silicone foam consisting of closed alveoli.

8. A prosthesis according to any one of the preceding claims characterised in that it comprises several portions which can be inflated or deflated independently.

9. A prosthesis according to any one of the preceding claims characterised in that it comprises a peripheral compartment which is provided with facets.

## Patentansprüche

1. Prothese umfassend eine peripherische Silikonmembran (1), die zumindest teilweise mit einer physiologischen Salzlösung gefüllt ist, dadurch gekennzeichnet, daß das Volumen, das von der Membran (1) begrenzt wird, mit einer Anzahl kleiner individueller geschlossener Zellen (5) ausgefüllt ist, wobei jede durch eine relativ dünne Silikonmembran begrenzt und mit einer biokompatiblen Flüssigkeit gefüllt ist, und die kleinen Zellen (5) um einen zentralen Sektor (4) verteilt sind, der durch eine geschlossene, relativ dicke Silikonmembran (6, 7) begrenzt ist, und wobei der Sektor mit einer konstant einstellbaren Menge der physiologischen Salzlösung gefüllt ist.

2. Prothese nach Anspruch 1, dadurch gekennzeichnet, daß die peripherische Membran (1) einen dickeren Anteil (3) umfaßt, der, nachdem er mit einer Nadel durchstochen wird, selbstschließend ist.

3. Prothese nach Anspruch 1 oder 2, dadurch gekennzeichnet, daß die peripherische Membran (1) einen oder mehrere versteifte Anteile (2) umfaßt.

4. Prothese nach einem der vorhergehenden Ansprüche, dadurch gekennzeichnet, daß die kleinen Zellen (5) ein Volumen von weniger als 1 cm³ besitzen.

5. Prothese nach einem der vorhergehenden Ansprüche, dadurch gekennzeichnet, daß die kleinen Zellen (5) und der zentrale Sektor (4) mit einem Silikonkleber beschichtet sind.

6. Prothese nach einem der vorhergehenden Ansprüche, dadurch gekennzeichnet, daß der zentrale Sektor (4) zwei Halbkugeln umfaßt, von denen eine aus einem selbstschließenden Silikonmaterial hergestellt ist, und die andere aus einem ausdehnbaren Silikonfilm.

7. Prothese nach einem der vorhergehenden Ansprüche, dadurch gekennzeichnet, daß das System der kleinen Zellen (5) einen Silikonschaum aus geschlossenen Alveolen umfaßt.

8. Prothese nach einem der vorhergehenden Ansprüche, dadurch gekennzeichnet, daß sie mehrere Anteile umfaßt, die unabhängig voneinander mit Gas aufgeblasen oder von Gas entleert werden können.

9. Prothese nach einem der vorhergehenden Ansprüche, dadurch gekennzeichnet, daß sie einen peripherischen Abschnitt umfaßt, der mit Facetten versehen ist.

## Revendications

1. Prothèse comprenant une membrane périphérique (1) en silicone remplie au moins partiellement par une solution saline physiologique, caractérisée en ce que le volume délimité par ladite membrane (1) est rempli par un certain nombre de petites cellules individuelles fermées (5), délimitées chacune par une membrane en silicone relativement mince et remplies par un fluide biocompatible, lesdites petites cellules (5) étant réparties autour d'un compartiment central (4) délimité par une membrane en silicone relativement épaisse (6, 7), ledit compartiment étant rempli par une quantité constamment ajustable de ladite solution physiologique.

2. Prothèse selon la revendication 1, caractérisé en ce que ladite membrane périphérique (1) comprend une portion plus épaisse (3) auto-cicatrisable après avoir été perforée par une aiguille.

3. Prothèse selon l'une des revendications 1 ou 2, caractérisé en ce que ladite membrane périphérique (1) comprend une ou plusieurs portions rigidifères (2).

4. Prothèse selon l'une quelconque des revendications précédentes, caractérisé en ce que les petites cellules (5) ont un volume inférieur à 1cm³.

5. Prothèse selon l'une quelconque des revendications précédentes, caractérisé en ce que les petites cellules (5) et le compartiment central (4) sont enduites d'un adhésif du type silicone.

6. Prothèse selon l'une quelconque des revendications précédentes, caractérisé en ce que le compartiment central (4) comprend deux hémisphères dont l'un est réalisé en un silicone auto-cicatrisable et l'autre est réalisé en un film de silicone extensible.

7. Prothèse selon l'une quelconque des revendications précédentes, caractérisé en ce que l'ensemble des petites cellules (5) est constitué par une mousse de silicone à alvéoles fermées.

8. Prothèse selon l'une quelconque des revendications précédentes, caractérisé en ce qu'elle comprend plusieurs portions pouvant être gonflées ou dégonflées indépendamment.

9. Prothèse selon l'une quelconque des revendications précédentes, caractérisé en ce qu'elle comprend un compartiment périphérique présentant des facettes.
